# EUROPÄISCHE PATENTSCHRIFT

(11) **EP 2 987 765 B1**
(45) Veröffentlichungstag und Bekanntmachung des Hinweises auf die Patenterteilung: **19.09.2018**
(21) Anmeldenummer: 15178674.6
(22) Anmeldetag: 28.07.2015
(51) Int. Cl.: B67B 7/92, B67B 7/00

(54) **GESPANNTE ÖFFNUNGSVORRICHTUNG FÜR MONOMERBEHÄLTER**
TENSIONED OPENING DEVICE FOR MONOMER CONTAINER
DISPOSITIF D'OUVERTURE PRECONTRAINT POUR RECIPIENT MONOMERE

(30) Priorität: 22.08.2014 DE 102014112043
(43) Veröffentlichungstag der Anmeldung: 24.02.2016
(73) Patentinhaber: Heraeus Medical GmbH, 61273 Wehrheim (DE)
(72) Erfinder: Dr. Vogt, Sebastian, 99092 Erfurt (DE); Dr. Kluge, Thomas, 56179 Vallendar (DE)
(74) Vertreter: Heraeus IP

(56) Entgegenhaltungen:
- EP-A2- 0 926 500
- GB-A- 1 496 724
- GB-A- 2 091 240
- US-A- 2 179 588
- US-A- 2 655 767

## Beschreibung

Die Erfindung betrifft eine Öffnungsvorrichtung zum Öffnen eines Monomerflüssigkeitsbehälters, der eine Ampulle ist.

Die Erfindung betrifft ferner ein Vakuummischsystem mit einer solchen Öffnungsvorrichtung und ein Verfahren zum Öffnen eines Monomerflüssigkeitsbehälters, der eine Ampulle ist.

Gegenstand der Erfindung ist somit eine Vorrichtung zum Öffnen von Monomerflüssigkeitsbehältern von Pulver-Flüssigkeits-Polymethylmethacrylat-Knochenzementen zur Herstellung derselben. Ein weiterer Gegenstand der Erfindung ist ein geschlossenes Vakuummischsystem für die Lagerung, Vermischung und den Austrag von Polymethylmethacrylat-Knochenzement.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden mit der EP 0 692 229 A1, der DE 10 2009 031 178 B3, der US 5,997,544 A, der US 6,709,149 B1, der DE 698 12 726 T2 und der US 5,588,745 A vorgeschlagen.

Das Patent DE 10 2009 031 178 B3 offenbart ein geschlossenes Vakuummischsystem mit einem zweiteiligen Austragskolben zum Verschluss einer Zement-Kartusche. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

Bei den bisher üblichen Vakuummischsystemen erfolgt die Öffnung des Monomerflüssigkeitsbehälters entweder durch manuelles Abdrehen des Ampullenkopfs, wie beispielsweise in der WO 2010/012114 A1 vorgeschlagen oder auch durch manuelles Aufstechen von Monomerflüssigkeitsbeuteln. Die Vermischung des Zementpulvers mit der Monomerflüssigkeit wird anschließend durch manuell zu betätigende Mischvorrichtungen vorgenommen. In der Zukunft, ist mit einem Bedarf an maximal vereinfachten Vakuummischsystemen zu rechnen. Diese sollen möglichst selbsttätig und weitgehend automatisch arbeiten.

GB 2 091 240 A offenbart eine Vorrichtung, gemäß dem Oberbegriff des Anspruchs 1, zum Öffnen von Ampullen, wobei eine Halterung für die Ampullen verschiebbar angeordnet ist, die Ampulle in der Halterung an einem Schneidgerät vorbeigeführt wird, das den Ampullenhals anritzt und eine Vorrichtung anschließend den Ampullenkopf abschlägt.

Die Aufgabe der Erfindung besteht also darin, die Nachteile des Stands der Technik zu überwinden. Insbesondere soll eine Öffnungsvorrichtung für einen Monomerflüssigkeitsbehälter, ein Vakuummischsystem und ein Verfahren zum Öffnen eines Monomerflüssigkeitsbehälters bereitgestellt werden, die eine möglichst stark vereinfachte Anwendung ermöglicht. Dabei soll der Monomerflüssigkeitsbehälter innerhalb eines geschlossenen Systems beziehungsweise eines geschlossenen oder verschließbaren Vakuummischsystems zu öffnen sein, ohne dass Teile oder Tröpfchen des Inhalts nach außen abgegeben werden können.

Eine Aufgabe der Erfindung ist dabei ferner darin zu sehen, eine Vorrichtung zur nicht manuellen Öffnung von Monomerflüssigkeitsbehältern zu entwickeln. Das bedeutet, die Vorrichtung soll nach Aktivierung eine sichere selbsttätige Öffnung des Monomerflüssigkeitsbehälters bewirken. Der Antrieb der Vorrichtung darf dabei nicht über externe Energiequellen angetrieben werden. Es soll eine einfache, lagerfähige Energiequelle eingesetzt werden, die in ein geschlossenes Vakuummischsystem integriert werden kann.

Des Weiteren soll ein geschlossenes Vakuummischsystem entwickelt werden, in der die Öffnungsvorrichtung zur Öffnung des Monomerflüssigkeitsbehälters integriert ist. Das Vakuummischsystem soll eine Zementkartusche enthalten, in der ein Zementpulver gelagert ist, sowie einen davon separaten Monomerflüssigkeitsbehälter, in dem sich die Monomerflüssigkeit befindet. Die Monomerflüssigkeit wird dadurch getrennt von dem Zementpulver gelagert. Vor und während der Vermischung der beiden Zementkomponenten - dem Zementpulver mit der Monomerflüssigkeit - soll ein Kontakt der medizinischen Anwender mit diesen Komponenten ausgeschlossen sein. Die Öffnung des Monomerflüssigkeitsbehälters und der Transfer der Monomerflüssigkeit sollen daher in einem geschlossenen System erfolgen. Das Zementpulver darf ebenfalls nicht in Kontakt zum medizinischen Anwender kommen.

Die Aufgaben der Erfindung werden gelöst durch eine Öffnungsvorrichtung zum Öffnen eines Monomerflüssigkeitsbehälters, der eine Ampulle ist, nach Anspruch 1, aufweisend:
a) eine Positionierhilfe zur Positionierung der Ampulle,
b) eine Öffnungseinrichtung zum Öffnen der Ampulle, wobei die Öffnungseinrichtung gegen die Positionierhilfe beweglich gelagert ist und/oder die Positionierhilfe gegen die Öffnungseinrichtung beweglich gelagert ist, und
c) zumindest ein elastisch deformierbares Energiespeicherelement zur Speicherung elastischer Energie, wobei eine Bewegung der Öffnungseinrichtung und/oder eine Bewegung der Positionierhilfe mit der elastischen Energie des zumindest einen elastisch deformierbaren Energiespeicherelements anzutreiben ist und die Ampulle durch die Bewegung der Positionierhilfe mit der Ampulle und der Öffnungseinrichtung relativ zueinander zu öffnen ist, wobei die Öffnungsvorrichtung ein Gefäß zur Aufnahme der Ampulle aufweist und das Gefäß einen Raum für die Aufnahme eines Ampullenkopfs der Ampulle aufweist, wobei dieser Raum mindestens eine mechanisch deformierbare Wand besitzt, an welcher der Ampullenkopf anliegt oder sich in nahem Abstand dazu befindet, wobei die durch die elastische Energie angetriebene Öffnungseinrichtung die mechanisch deformierbare Wand derart deformiert, dass der Ampullenkopf abbricht oder aufbricht.

Der Monomerflüssigkeitsbehälter kann Teil der Öffnungsvorrichtung sein, es kann aber auch vorgesehen sein, dass der Monomerflüssigkeitsbehälter in die Öffnungsvorrichtung eingesetzt werden muss, um ihn mit der Öffnungsvorrichtung zu öffnen. In dem Monomerflüssigkeitsbehälter ist bevorzugt eine Monomerflüssigkeit enthalten. Die Positionierhilfe dient lediglich dazu, den Monomerflüssigkeitsbehälter derart in einer Position zu halten oder zu drücken, dass er mit der Öffnungseinrichtung geöffnet werden kann, ohne dass sich der Monomerflüssigkeitsbehälter aufgrund der durch die Öffnungseinrichtung bewirkte Kraft derart deformiert oder bewegt, dass er nicht geöffnet wird. Die elastische Energie kann einfach zur unmittelbaren Bewegung der Öffnungseinrichtung und/oder des Monomerflüssigkeitsbehälters eingesetzt werden. Es ist auch möglich eine Übersetzung oder ein Gestänge oder ähnliches vorzusehen, um die elastische Kraft auf die Öffnungseinrichtung und/oder die Positionierhilfe zu vermitteln.

Bevorzugt wird die Öffnungseinrichtung gegen den Monomerflüssigkeitsbehälter gedrückt und öffnet diesen, wenn die Kraft, die durch das elastische Energiespeicherelement vermittelt wird, über die Öffnungseinrichtung auf den Monomerflüssigkeitsbehälter groß genug ist, um diesen abzubrechen oder aufzubrechen.

Bei erfindungsgemäßen Öffnungsvorrichtungen ist vorgesehen, dass die Öffnungsvorrichtung ein Gefäß zur Aufnahme des Monomerflüssigkeitsbehälters aufweist, insbesondere ein geschlossenes oder verschließbares Gefäß zur Aufnahme des Monomerflüssigkeitsbehälters aufweist, wobei bevorzugt die Positionierhilfe im Inneren des Gefäßes angeordnet ist, besonders bevorzugt an einer Innenwandung des Gefäßes angeordnet ist.

Mit einem solchen Gefäß kann sichergestellt werden, dass keine Monomerflüssigkeit aus dem geöffneten Monomerflüssigkeitsbehälter ungewollt in die Umgebung gelangen kann. Die Öffnungsvorrichtung und/oder das Energiespeicherelement können sich dabei auch außerhalb des Gefäßes und auch des geschlossenen oder verschließbaren Gefäßes befinden. Hierzu weist das Gefäß eine deformierbare Wandung auf, über die eine Kraft ins Innere des Gefäßes auf den Monomerflüssigkeitsbehälter weitergeleitet werden kann.

Ferner ist vorgesehen, dass der Monomerflüssigkeitsbehälter eine Ampulle ist, insbesondere eine Glasampulle ist, die mit der Positionierhilfe zu positionieren oder positioniert ist, insbesondere zu halten oder gehalten ist, wobei bevorzugt die Öffnungseinrichtung eine Brecheinrichtung ist, die zum Öffnen der Ampulle die Ampulle zerbricht oder aufbricht, besonders bevorzugt einen Ampullenkopf der Ampulle abbricht oder aufbricht.

Um das Aufbrechen oder Zerbrechen der Ampulle zu erleichtern, kann an der Ampulle wenigstens eine Sollbruchstelle vorgesehen sein. Die Brecheinrichtung kann als Stößel mit einem stumpfen Ende oder mit einer Kante ausgeführt sein. In solchen Ampullen kann die Monomerflüssigkeit besonders lange und dauerhaft gelagert werden.

Des Weiteren kann auch vorgesehen sein, dass am Gefäß ein Ampullenhalter angeordnet ist, mit dem die Ampulle fixiert ist oder fixierbar ist.

Hierdurch kann der Aufbau kompakt gestaltet werden und die Stabilität des Gefäßes kann zur Halterung und Stabilisierung des Ampullenhalters eingesetzt werden.

Bei einer erfindungsgemäßen Öffnungsvorrichtung ist vorgesehen, dass das Gefäß einen Raum für die Aufnahme eines Ampullenkopfs der Ampulle aufweist, wobei dieser Raum mindestens eine mechanisch deformierbare Wand besitzt, an welcher der Ampullenkopf anliegt oder sich in nahem Abstand dazu befindet, wobei die durch die elastische Energie angetriebene Öffnungseinrichtung die mechanisch deformierbare Wand derart deformiert, dass der Ampullenkopf abbricht oder aufbricht, wobei bevorzugt mindestens ein für Flüssigkeiten durchlässiges Siebelement oder Filterelement vorgesehen ist, das im Raum zur Zurückhaltung des Ampullenkopfs und/oder von Bruchstücken der Ampulle unterhalb des Ampullenkopfs angeordnet ist.

Der Ampullenkopf befindet sich im nahen Abstand zu der elastische deformierbaren Wand, wenn die Auslenkung der deformierbaren Wand, die durch die Öffnungseinrichtung bewirkt wird, ausreicht, um den Ampullenkopf abzubrechen oder aufzubrechen. Bevorzugt ist die der Ampullenkopf nicht mehr als 25 mm von der elastischen Wand beabstandet, besonders bevorzugt nicht mehr als 10 mm von der elastischen Wand beabstandet, ganz besonders bevorzugt nicht mehr als 5 mm von der elastischen Wand beabstandet. Hierdurch wird ein vollkommen geschlossenes System bereitgestellt, bei dem die Monomerflüssigkeit nur mit dem Inneren des Gefäßes in Kontakt kommt und ein Austreten von Monomerflüssigkeit zuverlässig vermieden werden kann. Durch das Auffangen abgebrochener Bruchstücke der Ampulle werden störende Splitter ferngehalten. Hierdurch kann auch eine Glasampulle gefahrlos als Monomerflüssigkeitsbehälter eingesetzt werden.

Mit einer Weiterbildung der vorliegenden Erfindung wird vorgeschlagen, dass die Öffnungseinrichtung ein linear beweglich gelagerter Stößel ist und die lineare Bewegung des Stößels mit der elastischen Energie des zumindest einen elastisch deformierbaren Energiespeicherelements anzutreiben ist.

Mit dem Stößel lässt sich ein aufbrechbarer Monomerflüssigkeitsbehälter einfach öffnen. Der Stößel lässt sich besonders gut mit einer Feder als elastisches Energiespeicherelement antreiben.

Bevorzugte erfindungsgemäße Öffnungsvorrichtungen können vorsehen, dass das zumindest eine elastisch deformierbare Energiespeicherelement zumindest eine Feder ist, bevorzugt zumindest eine Metallfeder, besonders bevorzugt ausgewählt aus Stahlschenkelfedern, Stahlblattfedern und Stahlspiralfedern.

Federn sind sehr gut als elastische Energiespeicher geeignet, da sie sich leicht fertigen lassen, beziehungsweise als Massenprodukt kostengünstig zu erhalten sind, und die in ihnen gespeicherte Energie zum Öffnen des Monomerflüssigkeitsbehälters ausreicht. Bei der Verwendung einer Glasampulle als Monomerflüssigkeitsbehälter wird die Verwendung von Metallfedern bevorzugt, um die etwas höhere notwendige elastische Energie durch die höhere Federkraft solcher Metallfedern zu erhalten, die zum zuverlässigen Aufbrechen oder Zerbrechen der Glasampulle notwendig ist. Gegebenenfalls muss auch die Energie zum Deformieren einer elastischen Wand des Gefäßes zusätzlich mit aufgebracht werden können und daher ist die Feder bezüglich ihrer Federkraft entsprechend zu dimensionieren. Die Feder ist bevorzugt eine Spiralfeder. Anstatt einer normalen Feder kann auch ein Gummiband, eine Gasdruckfeder, eine hydraulische Federung oder irgendein anderer elastisch deformierbarer Energiespeicher verwendet werden.

Dabei kann vorgesehen sein, dass die zumindest eine Feder mit einer Federführung geführt ist, so dass die elastische Federkraft der zumindest einen Feder in eine durch die Federführung vorgegebene Richtung wirkt.

Hierdurch kann eine zuverlässige Öffnung des Monomerflüssigkeitsbehälters erreicht werden, da so sichergestellt ist, dass die Federkraft immer in die gewünschte Richtung wirkt.

Mit einer Weiterbildung der vorliegenden Erfindung wird vorgeschlagen, dass das zumindest eine elastisch deformierbare Energiespeicherelement gespannt ist und im gespannten Zustand durch mindestens eine lösbare mechanische Sicherung blockiert ist, so dass eine Abgabe der elastischen Energie des zumindest einen Energiespeicherelements durch die mindestens eine Sicherung verhindert ist, wobei bevorzugt die mindestens eine Sicherung als Sperrklinke und/oder als Sperrstift ausgebildet ist.

Mit der zumindest einen lösbaren mechanischen Sicherung kann die Öffnungsvorrichtung einfach bedient und eingesetzt werden. Bei Lösen der zumindest einen Sicherung wird die elastische Energie aus dem zumindest einen elastischen Energiespeicherelement freigesetzt und der Monomerflüssigkeitsbehälter geöffnet. Das Lösen der zumindest einen Sicherung kann manuell erfolgen und/oder mit anderen Sicherungen gekoppelt sein und/oder vollautomatisch erfolgen.

Zur leichteren Anwendbarkeit der Öffnungsvorrichtung mit Monomerflüssigkeiten kann auch vorgesehen sein, dass unterhalb der Positionierhilfe eine wannenförmige Aufnahme für die Monomerflüssigkeit vorgesehen ist.

Mit der wannenförmigen Aufnahme kann die Monomerflüssigkeit aufgefangen und dann genutzt werden.

Die der vorliegenden Erfindung zugrundliegenden Aufgaben werden auch gelöst durch ein Vakuummischsystem zum Herstellen eines Zements aufweisend eine erfindungsgemäße Öffnungsvorrichtung, eine Mischkartusche, in der ein Zementpulver enthalten ist, einen Monomerflüssigkeitsbehälter, der eine Ampulle ist und der durch die Positionierhilfe positioniert ist, und ein Leitungsmittel, das die Mischkartusche mit einer Öffnung im Bereich des Monomerflüssigkeitsbehälters oder der wannenförmigen Aufnahme für die Monomerflüssigkeit verbindet.

Das derart gestaltete Vakuummischsystem weist die Vorteile des Monomerflüssigkeitsbehälters auf. Diese sind aufgrund ihrer einfachen Bedienung und der Ungefälligkeit für Störungen insbesondere bei Vakuummischsystemen besonders vorteilhaft. Bevorzugt ist das Vakuummischsystem zum Mischen von PMMA-Zement unter Vakuum geeignet.

Dabei kann vorgesehen sein, dass das Vakuummischsystem einen Fußteil aufweist, der die Mischkartusche, das Leitungsmittel und die Öffnungsvorrichtung trägt und miteinander verbindet, wobei bevorzugt die Mischkartusche lösbar mit dem Fußteil verbunden ist.

Hierdurch kann das Vakuummischsystem einfach aufgebaut und aufgestellt werden. Gleichzeitig wird die für die Öffnungsvorrichtung notwendige Stabilität erreicht.

Die der Erfindung zugrundeliegenden Aufgaben werden aber auch gelöst durch ein Verfahren zum Öffnen eines Monomerflüssigkeitsbehälters mit einer vorab beschriebenen Öffnungsvorrichtung, bei dem die elastische Energie aus dem zumindest einen elastisch deformierten Energiespeicherelement entnommen wird, wobei mit der elastischen Energie die Öffnungseinrichtung und/oder die Positionierhilfe angetrieben wird, so dass die Öffnungseinrichtung und/oder die Positionierhilfe eine Kraft auf den Monomerflüssigkeitsbehälter ausüben, und mit der derart ausgeübten Kraft der Monomerflüssigkeitsbehälter geöffnet wird.

Dabei kann vorgesehen sein, dass zumindest ein Sicherungselement das zumindest eine Energiespeicherelement im elastisch deformierten Zustand hält, wobei das zumindest eine Sicherungselement gelöst wird, um die elastische Energie aus dem zumindest einen Energiespeicherelement zu entnehmen.

Ferner kann vorgesehen sein, dass eine gespannte Feder als Energiespeicherelement auf einen Stößel als Öffnungseinrichtung drückt und/oder eine gespannte Feder als Energiespeicherelement auf die Positionierhilfe oder auf eine Ampulle als Monomerflüssigkeitsbehälter drückt, wobei mit der Feder der Stößel gegen die Ampulle gedrückt wird und/oder die Ampulle gegen den Stößel gedrückt wird, wobei die Kraft, die dadurch auf die Ampulle ausgeübt wird, dazu verwendet wird, um die Ampulle zu öffnen, insbesondere um die Ampulle aufzubrechen oder zu zerbrechen.

Der Erfindung liegt die überraschende Erkenntnis zugrunde, dass es durch es durch die Entspannung eines elastisch verformten Energiespeicherelements, das eine Öffnungseinrichtung antreibt, gelingt, eine Öffnungsvorrichtung und ein Vakuummischsystem für PMMA-Knochenzemente sowie ein Verfahren zum Öffnen eines Monomerflüssigkeitsbehälters bereitzustellen, bei dem kein manuelles Öffnen des Monomerflüssigkeitsbehälters notwendig ist und in dem bereits die für die Öffnung des Monomerflüssigkeitsbehälters notwendige Energie gespeichert ist. Mit der Öffnungseinrichtung und dem elastischen Energiespeicher kann eine zuverlässige und schnelle Öffnung des Monomerflüssigkeitsbehälters erfolgen, ohne dass eine zu starke oder eine zu schwache Krafteinwirkung das Öffnen des Monomerflüssigkeitsbehälters behindern würde. Eine externe Energiequelle ist dadurch überflüssig. Die erfindungsgemäßen Öffnungsvorrichtungen und das erfindungsgemäße Verfahren kommen dabei vollständig ohne elektrische Antriebe oder Motoren aus. Die Anwendung ist deutlich vereinfacht, was sich insbesondere bei oft hektischen Vorgängen während einer Operation (OP) positiv auswirkt. Der Aufbau bleibt dabei kostengünstig und das Produkt kann daher auch als Einmalartikel angeboten werden, was für die hygienischen Anforderungen im OP-Bereich sinnvoll ist. Mit erfindungsgemäßen Verfahren und Öffnungsvorrichtungen kann ein PMMA-Knochenzement auf einfache Weise hergestellt werden. Das erfindungsgemäße Vakuummischsystem ist zudem zur Lagerung der Ausgangskomponenten und zur Applikation des fertig gemischten Knochenzements geeignet.

Neue Vakuummischsysteme, wie das erfindungsgemäße Vakuummischsystem, sollen möglichst selbsttätig und weitgehend automatisch arbeiten. Für ein selbsttätiges automatisches Mischsystem ist es am besten, wenn die Öffnungsvorrichtung beziehungsweise das erfindungsgemäße Vakuummischsystem nach Aktivierung selbsttätig den oder die Monomerbehälter öffnet. Dies wird mit der vorliegenden erfindungsgemäßen Öffnungsvorrichtung und dem erfindungsgemäßen Vakuummischsystem erreicht.

Erfindungsgemäße Öffnungsvorrichtungen zum Öffnen von Monomerflüssigkeitsbehältern sind dadurch charakterisiert, dass zur Öffnung von Monomerflüssigkeitsbehältern mindestens ein elastisch, deformierbares Energiespeicherelement als Energiequelle verwendet wird.

Im gespannten Zustand ist das Energiespeicherelement bevorzugt durch mindestens eine lösbare mechanische Sicherung blockiert, durch die eine Abgabe der Energie des Energiespeicherelements verhindert wird, wobei diese Sicherung bevorzugt als Sperrklinke und/oder als Sperrstift ausgebildet ist.

Eine beispielhafte bevorzugte Ausführungsform der erfindungsgemäßen Öffnungsvorrichtung weist die Folgenden Teile auf:
a) einen Ampullenhalter mit einer Monomerflüssigkeitsampulle,
b) einen Raum für die Aufnahme des Ampullenkopfs, wobei dieser Raum mindestens eine mechanisch deformierbare Wand besitzt, an welcher der Ampullenkopf anliegt oder sich in nahem Abstand dazu befindet,
c) mindestens ein für Flüssigkeiten durchlässiges Siebelement, das im Raum zur Aufnahme des Ampullenkopfs unterhalb des Ampullenkopfs angeordnet ist,
d) unterhalb des Siebelements eine wannenförmige Aufnahme für die Monomerflüssigkeit,
e) mindestens ein Stößel mit einer Federaufnahme,
f) mindestens eine Feder mit Federführung und
g) mindestens ein Sicherungselement, das den Stößel bei gespannter Feder vor der Wand der Aufnahme des Ampullenkopfs fixiert.

Bei dieser Ausführung dient die Monomerflüssigkeitsampulle als Monomerflüssigkeitsbehälter, der Ampullenhalter als Positionierhilfe, die Feder als elastisches Energiespeicherelement oder als eines der zumindest zwei Energiespeicherelemente und der Stößel als Öffnungseinrichtung.

Erfindungsgemäß ist beispielsweise ein Verfahren zum Öffnen eines Monomerflüssigkeitsbehälters mit der erfindungsgemäßen Öffnungsvorrichtung. Dieses Verfahren kann beispielsweise dadurch charakterisiert sein, dass
a) zuerst das Sicherungselement gelöst wird,
b) dann die Feder den Stößel auf die deformierbare Wand des Raums zur Aufnahme des Ampullenkopfs bewegt,
c) die Wand durch Einwirkung des Stößels in Richtung des Ampullenkopfs deformiert wird,
d) die deformierte Wand den Ampullenkopf abbricht, und
e) die Monomerflüssigkeit aus der Ampulle ausläuft und durch das Siebelement in die Aufnahme für die Monomerflüssigkeit zur Weiterleitung in die Mischkartusche fließt.

Im Folgenden werden weitere Ausführungsbeispiele der Erfindung anhand von sieben schematisch dargestellten Figuren erläutert, ohne jedoch dabei die Erfindung zu beschränken. Dabei zeigt:
Figur 1: eine schematische Querschnittansicht einer erfindungsgemäßen Öffnungsvorrichtung im gesicherten Zustand;
Figur 2: eine schematische Querschnittansicht der erfindungsgemäßen Öffnungsvorrichtung nach Figur 1 im entsicherten Zustand.

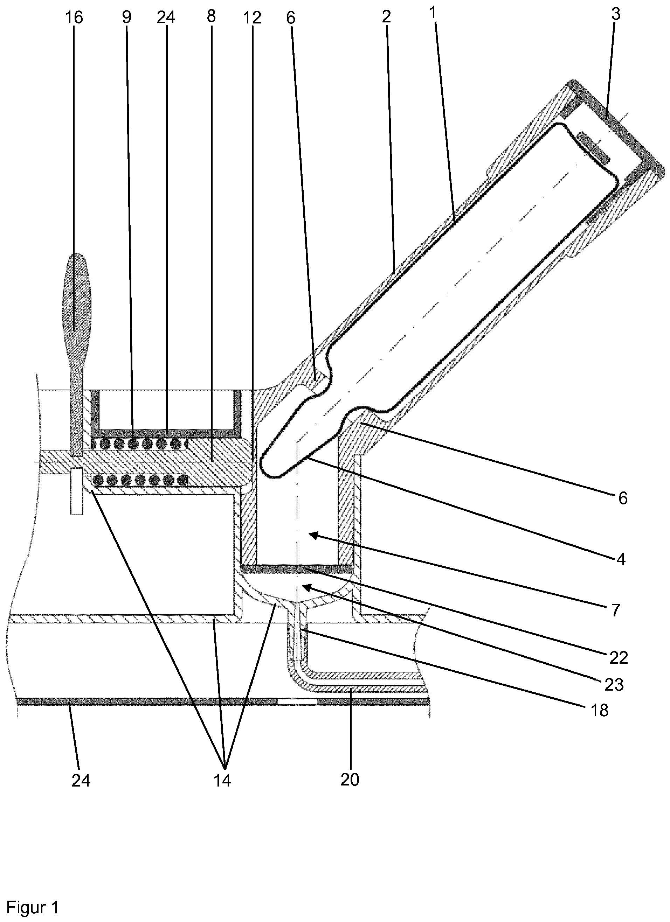

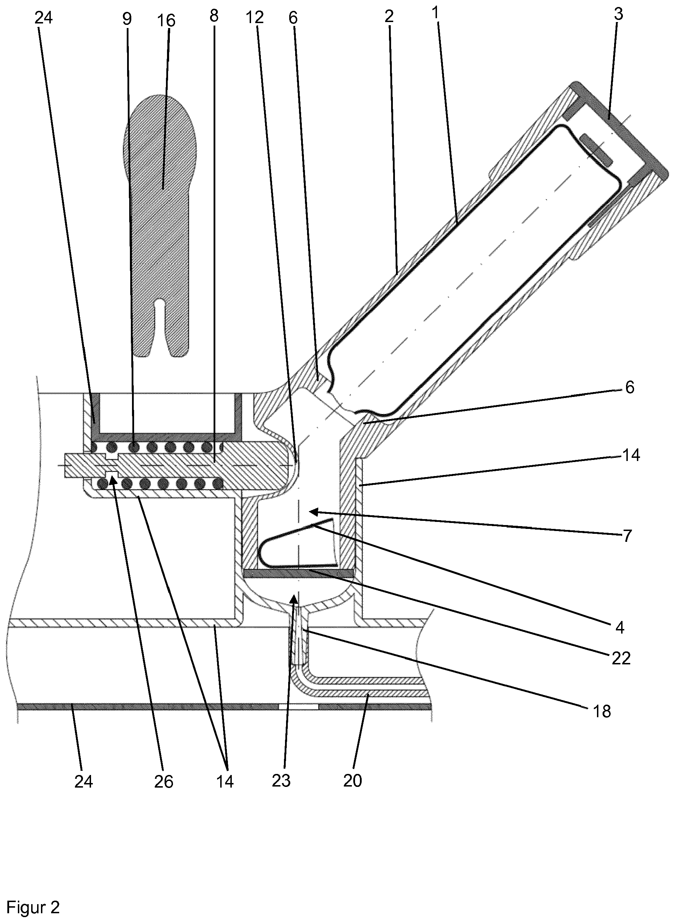

Die Figuren 1 und 2 zeigen schematische Querschnittansichten einer erfindungsgemäßen Öffnungsvorrichtung im gesicherten Zustand (Figur 1) und im entsicherten Zustand (Figur 2) als Teil eines Vakuummischsystems.

In der Öffnungsvorrichtung ist eine Glasampulle 1 angeordnet, die mit der Öffnungsvorrichtung zu öffnen ist. Die Glasampulle 1 enthält ein flüssiges Monomer als Ausgangskomponente zur Herstellung eines PMMA-Zements. Die Glasampulle 1 ist also ein Monomerflüssigkeitsbehälter 1. Zum Öffnen ist die Glasampulle 1 in ein Gefäß 2 eingesteckt, das mit einem Deckel 3 nach Art eines Stopfens fluiddicht verschlossen ist. Ein Ampullenkopf 4 der Glasampulle 1 ist durch eine Halterung 6 hindurchgesteckt, die durch eine Verdickung des Materials des Gefäßes 2 ausgebildet ist. Mit der Halterung 6 aber auch mit dem Aufbau des Gefäßes 2 und des Deckels 3 wird die Glasampulle 1 innerhalb des Gefäßes 2 fixiert. Der Ampullenkopf 4 wird zum Öffnen der Glasampulle 1 aufgebrochen beziehungsweise abgebrochen. Unterhalb des Ampullenkopfs 4 ist in dem Gefäß 2 ein Raum 7 zum Auffangen des abgebrochenen Ampullenkopfs 4 vorgesehen.

Der Ampullenkopf 4 kann mit einem Stößel 8 abgebrochen werden, der mit einer Feder 9 aus Metall oder einem Kunststoff in Richtung des Ampullenkopfs 4 vorgetrieben werden kann. Der Stößel 8 und die Feder 9 sind außerhalb des Gefäßes 2 angeordnet. Damit die zum Abbrechen des Ampullenkopfs 4 notwendige Kraft vom Stößel 8 auf den Ampullenkopf 4 übertragen werden kann, ist eine deformierbare Wandung 12 am Gefäß 2 zwischen dem Stößel 8 und dem Ampullenkopf 4 beziehungsweise der Glasampulle 1 vorgesehen. Die Wandung 12 ist vorliegend dadurch deformierbar, dass die eine geringere Dicke aufweist. Wenn der Stößel 8 von der Feder 9 in Richtung des Ampullenkopfs 4 vorgetrieben wird, deformiert sich die Wandung 12 und der Ampullenkopf 4 wird vom Körper der Glasampulle 1 abgebrochen und damit geöffnet. Der abgebrochene Ampullenkopf 4 fällt in den Raum 7 und der Inhalt der Glasampulle 1 (die Monomerflüssigkeit) ergießt sich aus der Ampulle 1.

Die Feder 9 ist gegen einen Vorsprung des Stößels 8 und gegen ein Gerüst 14 aus Kunststoff gelagert, wobei das Gerüst 14 den Grundkörper der Öffnungsvorrichtung bildet. Die Feder 9 ist in Figur 1 gespannt. Die Feder 9 und der Stößel 8 sind in Figur 1 gesichert, indem ein Sicherungsstift 16 als Sicherung 16 in eine Ausnehmung 26 im Stößel 8 eingesteckt ist, so dass der Sicherungsstift 16 eine Bewegung des Stößels 8 blockiert und der Sicherungsstift 16 von dem Stößel 8 an das Gerüst 14 gezogen wird.

Der Raum 7 mündet in einen Stutzen 18 in Form eines Rohrstücks, auf das eine Leitung 20 in Form eines Schlauchs 20 aufgesteckt ist. Die Leitung 20 ist bei Vakuummischsystemen mit einer Mischkartusche (nicht gezeigt) verbunden, in der ein Zementpulver angeordnet ist. Die Monomerflüssigkeit aus der geöffneten Glasampulle 1 kann durch diese Leitung 20 zur Mischkartusche geleitet werden, indem ein Vakuum in der Mischkartusche angelegt wird, dass die Monomerflüssigkeit aus dem Gefäß 2 durch die Leitung 20 in die Mischkartusche einsaugt. Die Monomerflüssigkeit kann dann mit Hilfe einer Mischvorrichtung (nicht gezeigt) in der Mischkartusche unter Vakuum mit dem Zementpulver aus der Mischkartusche gemischt werden, um einen PMMA-Zement herzustellen.

Damit der abgebrochene Ampullenkopf 4 nicht die Zuleitung durch den Rohrstutzen 18 blockieren kann und damit keine Bruchstücke des Glases der Glasampulle 1 in den zu mischenden PMMA-Zement gelangen können, ist unterhalb des Ampullenkopfs 4 ein Sieb 22 oder ein Filter 22 vorgesehen. Unterhalb des Siebs 22 oder Filters 22 ist der Raum 7 als Auffangwanne 23 nach Art eines Trichters geformt, um die Monomerflüssigkeit aufzufangen und in den Rohrstutzen 18 zu leiten.

Das Vakuummischsystem weist einen Standfuß 24 beziehungsweise ein Fußteil 24 auf, das mit dem Gerüst 14 fest verbunden ist oder das Gerüst 14 ist ein Teil des Fußteils 24. Das Fußteil 24 dient der Aufstellung des Vakuummischsystems und hält die Öffnungsvorrichtung mit der Glasampulle 1 und auch die Mischkartusche und die Leitung 20.

Die Monomerflüssigkeit kann in der geschlossenen Glasampulle 1 problemlos auch über längere Zeiträume gelagert werden. Die Öffnung der Glasampulle 1 erfolgt kurz vor dem Mischen mit dem Zementpulver. Zum Öffnen der Glasampulle 1 muss nur einfach der Sicherungsstift 16 aus der Öffnungsvorrichtung beziehungsweise dem Fußteil 24 herausgezogen werden. Die Feder 9 treibt dann den Stößel 8 an. Der in Richtung des Ampullenkopfs 4 vorgetriebene Stößel 8 deformiert die Wandung 12 und bricht den Ampullenkopf vom Ampullenkörper der Glasampulle 1 ab. Die Monomerflüssigkeit ergießt sich aus der Glasampulle 1 durch den Filter 22 oder das Sieb 22 hindurch und sammelt sich in der Auffangwanne 23. Von dort aus kann die Monomerflüssigkeit mit einem Vakuum in der Mischkartusche durch den Rohrstutzen 18 und die Leitung 20 in die Mischkartusche gesaugt werden. Dort kann dann die Monomerflüssigkeit mit dem Zementpulver in der Mischkartusche gemischt werden, um den PMMA-Zement herzustellen beziehungsweise zu mischen.

Der Stößel 8 bildet auch eine Federführung für die Feder 9. Das Gerüst 14 und das Fußteil 24 können ebenfalls zur Führung der Feder 9 beitragen.

### Bezugszeichenliste

- 1: Glasampulle / Monomerflüssigkeitsbehälter
- 2: Gefäß
- 3: Deckel
- 4: Ampullenkopf
- 6: Halterung
- 7: Raum zum Auffangen des Ampullenkopfs
- 8: Stößel / Öffnungseinrichtung
- 9: Feder / elastisches Energiespeicherelement
- 12: Deformierbare Wandung
- 14: Gerüst
- 16: Sicherungsstift / Sicherung
- 18: Rohrstutzen
- 20: Leitung / Leitungsmittel
- 22: Sieb / Filter
- 23: Auffangwanne
- 24: Standfuß / Fußteil
- 26: Ausnehmung

## Patentansprüche

1. Öffnungsvorrichtung zum Öffnen eines Monomerflüssigkeitsbehälters, der eine Ampulle (1) ist, aufweisend
eine Positionierhilfe (6) zur Positionierung der Ampulle (1),
eine Öffnungseinrichtung (8) zum Öffnen der Ampulle (1),
wobei die Öffnungseinrichtung (8) gegen die Positionierhilfe (6) beweglich gelagert ist und/oder die Positionierhilfe (6) gegen die Öffnungseinrichtung (8) beweglich gelagert ist, und
zumindest ein elastisch deformierbares Energiespeicherelement (9) zur Speicherung elastischer Energie, wobei eine Bewegung der Öffnungseinrichtung (8) und/oder eine Bewegung der Positionierhilfe (6) mit der elastischen Energie des zumindest einen elastisch deformierbaren Energiespeicherelements (9) anzutreiben ist und die Ampulle (1) durch die Bewegung der Positionierhilfe (6) mit der Ampulle (1) und der Öffnungseinrichtung (8) relativ zueinander zu öffnen ist, wobei die Öffnungsvorrichtung ein Gefäß (2) zur Aufnahme der Ampulle (1) aufweist und das Gefäß (2) einen Raum (7) für die Aufnahme eines Ampullenkopfs (4) der Ampulle (1) aufweist, **dadurch gekennzeichnet, dass** dieser Raum (7) mindestens
eine mechanisch deformierbare Wand (12) besitzt, an welcher der Ampullenkopf (4) anliegt oder sich in nahem Abstand dazu befindet, wobei die durch die elastische Energie angetriebene Öffnungseinrichtung (8) die mechanisch deformierbare Wand (12) derart deformiert, dass der Ampullenkopf (4) abbricht oder aufbricht.

2. Öffnungsvorrichtung nach Anspruch 1, **dadurch gekennzeichnet, dass** die Positionierhilfe (6) im Inneren des Gefäßes (2) angeordnet ist.

3. Öffnungsvorrichtung nach einem der vorhergehenden Ansprüche, **dadurch gekennzeichnet, dass**
die Positionierhilfe (6) ein Ampullenhalter ist, der am Gefäß (2) angeordnet ist und mit dem die Ampulle (1) fixiert ist oder fixierbar ist.

4. Öffnungsvorrichtung nach einem der vorhergehenden Ansprüche, **dadurch gekennzeichnet, dass**
mindestens ein für Flüssigkeiten durchlässiges Siebelement (22) oder Filterelement (22) vorgesehen ist, das im Raum (7) zur Zurückhaltung des Ampullenkopfs (4) und/oder von Bruchstücken der Ampulle (1) unterhalb des Ampullenkopfs (4) angeordnet ist.

5. Öffnungsvorrichtung nach einem der vorgehenden Ansprüche, **dadurch gekennzeichnet, dass**
die Öffnungseinrichtung (8) ein linear beweglich gelagerter Stößel (8) ist und die lineare Bewegung des Stößels (8) mit der elastischen Energie des zumindest einen elastisch deformierbaren Energiespeicherelements (9) anzutreiben ist.

6. Öffnungsvorrichtung nach einem der vorangehenden Ansprüche, **dadurch gekennzeichnet, dass**
das zumindest eine elastisch deformierbare Energiespeicherelement (9) zumindest eine Feder (9) ist.

7. Öffnungsvorrichtung nach Anspruch 6, **dadurch gekennzeichnet, dass**
die zumindest eine Feder (9) mit einer Federführung geführt ist, so dass die elastische Federkraft der zumindest einen Feder (9) in eine durch die Federführung vorgegebene Richtung wirkt.

8. Öffnungsvorrichtung nach einem der vorangehenden Ansprüche, **dadurch gekennzeichnet, dass**
das zumindest eine elastisch deformierbare Energiespeicherelement (9) gespannt ist und im gespannten Zustand durch mindestens eine lösbare mechanische Sicherung (16) blockiert ist, so dass eine Abgabe der elastischen Energie des zumindest einen Energiespeicherelements (9) durch die mindestens eine Sicherung (16) verhindert ist.

9. Öffnungsvorrichtung nach einem der vorangehenden Ansprüche, **dadurch gekennzeichnet, dass**
unterhalb der Positionierhilfe (6) eine wannenförmige Aufnahme (23) für die Monomerflüssigkeit vorgesehen ist.

10. Vakuummischsystem zum Herstellen eines Zements aufweisend eine Öffnungsvorrichtung nach einem der Ansprüche 1 bis 9, eine Mischkartusche, in der ein Zementpulver enthalten ist, einen Monomerflüssigkeitsbehälter (1), der eine Ampulle ist und der durch die Positionierhilfe (6) positioniert ist, und ein Leitungsmittel (20), das die Mischkartusche mit einer Öffnung im Bereich der Ampulle (1) oder der wannenförmigen Aufnahme (23) für die Monomerflüssigkeit verbindet.

11. Vakuummischsystem nach Anspruch 10, **dadurch gekennzeichnet, dass**
das Vakuummischsystem einen Fußteil (24) aufweist, der die Mischkartusche, das Leitungsmittel (20) und die Öffnungsvorrichtung trägt und miteinander verbindet, wobei bevorzugt die Mischkartusche lösbar mit dem Fußteil (24) verbunden ist.

12. Verfahren zum Öffnen eines Monomerflüssigkeitsbehälters (1), der eine Ampulle ist, mit einer Öffnungsvorrichtung nach einem der Ansprüche 1 bis 10, wobei elastische Energie aus dem zumindest einen elastisch deformierten Energiespeicherelement (9) entnommen wird, wobei mit der elastischen Energie die Öffnungseinrichtung (8) und/oder die Positionierhilfe (6) angetrieben wird, so dass die Öffnungseinrichtung (8) und/oder die Positionierhilfe (6) eine Kraft auf die Ampulle (1) ausüben, und mit der derart ausgeübten Kraft die Ampulle (1) geöffnet wird.

13. Verfahren nach Anspruch 12, **dadurch gekennzeichnet, dass**
zumindest ein Sicherungselement (16) das zumindest eine Energiespeicherelement (9) im elastisch deformierten Zustand hält, wobei das zumindest eine Sicherungselement (16) gelöst wird, um die elastische Energie aus dem zumindest einen Energiespeicherelement (9) zu entnehmen.

14. Verfahren nach einem der Ansprüche 12 oder 13, **dadurch gekennzeichnet, dass** eine gespannte Feder (9) als Energiespeicherelement (9) auf einen Stößel (8) als Öffnungseinrichtung (8) drückt und/oder eine gespannte Feder (9) als Energiespeicherelement (9) auf die Positionierhilfe (6) oder auf die Ampulle (1) als Monomerflüssigkeitsbehälter (1) drückt, wobei mit der Feder (9) der Stößel (8) gegen die Ampulle (1) gedrückt wird und/oder die Ampulle (1) gegen den Stößel (8) gedrückt wird, wobei die Kraft, die dadurch auf die Ampulle (1) ausgeübt wird, dazu verwendet wird, um die Ampulle (1) zu öffnen.

## Claims

1. An opening device for opening a monomer liquid container which is an ampoule (1), comprising:
a positioning aid (6) for positioning the ampoule (1),
an opening feature (8) for opening the ampoule (1), wherein the opening feature (8) is movably mounted against the positioning aid (6) and/or the positioning aid (6) is movably mounted against the opening feature (8), and
at least one elastically deformable energy storage element (9) for storing elastic energy, wherein a movement of the opening feature (8) and/or a movement of the positioning aid (6) are/is to be driven by the elastic energy of the at least one elastically deformable energy storage element (9), and the ampoule (1) is to be opened by the movement of the positioning aid (6) with the ampoule (1) and the opening feature (8) relative to each other, wherein the opening device has a vessel (2) for receiving the ampoule (1) and the vessel (2) has a chamber (7) for receiving an ampoule head (4) of the ampoule (1), **characterised in that** this chamber (7) has at least one mechanically deformable wall (12), with the ampoule head (4) resting against and being located at a short distance from said wall, wherein the opening feature (8) that is driven by the elastic energy deforms the mechanically deformable wall (12) such that the ampoule head (4) breaks off or breaks open.

2. The opening device in accordance with Claim 1, **characterised in that** the positioning aid (6) is arranged inside the vessel (2).

3. The opening device in accordance with any one of the preceding claims, **characterised in that**
the positioning aid (6) is an ampoule holder which is arranged at the vessel (2) and with which the ampoule (1) is fixed or can be fixed.

4. The opening device in accordance with any one of the preceding claims, **characterised in that**
at least one sieve element (22) or filter element (22) that is permeable to liquids is provided, said element being arranged in the chamber (7) below the ampoule head (4) for the purpose of retaining the ampoule head (4) and/or fragments of the ampoule (1).

5. The opening device in accordance with any one of the preceding claims, **characterised in that**
the opening feature (8) is a tappet (8) that is mounted in a linearly movable manner and that the linear movement of the tappet (8) is to be driven by the elastic energy of the at least one elastically deformable energy storage element (9).

6. The opening device in accordance with any one of the preceding claims, **characterised in that**
the at least one elastically deformable energy storage element (9) is at least a spring (9).

7. The opening device in accordance with Claim 6, **characterised in that**
the at least one spring (9) is guided by a spring guide, with the result that the elastic spring force of the at least one spring (9) acts in a direction defined by the spring guide.

8. The opening device in accordance with any one of the preceding claims, **characterised in that**
the at least one elastically deformable energy storage element (9) is tensioned and, in the tensioned state, is blocked by at least one releasable mechanical securing device (16), with the result that a release of the elastic energy of the at least one energy storage element (9) is prevented by the at least one securing device (16).

9. The opening device in accordance with any one of the preceding claims, **characterised in that**
a tub-shaped receptacle (23) for the monomer liquid is provided below the positioning aid (6).

10. A vacuum mixing system for producing a cement, comprising an opening device in accordance with any one of Claims 1 to 9, a mixing cartridge in which a cement powder is contained, a monomer liquid container (1) which is an ampoule and which is positioned by means of the positioning aid (6), and a pipe means (20) which connects the mixing cartridge to an opening in the vicinity of the ampoule (1) or the tub-shaped receptacle (23) for the monomer liquid.

11. The vacuum mixing system in accordance with Claim 10, **characterised in that** the vacuum mixing system has a foot part (24) which carries the mixing cartridge, the pipe means (20) and the opening device and connects the same to each other, wherein the mixing cartridge is preferably connected to the foot part (24) in a releasable manner.

12. A method for opening a monomer liquid container (1) which is an ampoule having an opening device in accordance with any one of Claims 1 to 10, wherein the elastic energy is taken from the at least one elastically deformable energy storage element (9), wherein the opening feature (8) and/or the positioning aid (6) are/is driven by the elastic energy, with the result that the opening feature (8) and/or the positioning aid (6) exert(s) a force onto the ampoule and the ampoule (1) is opened by the force exerted in such a manner.

13. The method in accordance with Claim 12, **characterised in that** at least one securing element (16) keeps the at least one energy storage element (9) in its elastically deformable state, wherein the at least one securing element (16) is released to take the elastic energy from the at least one energy storage element (9).

14. The method in accordance with any one of Claims 12 or 13, **characterised in that** a tensioned spring (9), being the energy storage element (9), presses onto a tappet (8) being an opening feature (8), and/or a tensioned spring (9), being the energy storage element (9), presses onto the positioning aid (6) or onto the ampoule (1) being the monomer liquid container (1), wherein the tappet (8) is pressed against the ampoule (1) by means of the spring (9) and/or the ampoule (1) is pressed against the tappet (8), wherein the force that is thereby exerted on the ampoule (1) is used to open the ampoule (1).

## Revendications

1. Dispositif d'ouverture pour l'ouverture d'un récipient de liquide monomère qui est une ampoule (1), présentant
une aide au positionnement (6) pour le positionnement de l'ampoule (1),
un appareil d'ouverture (8) pour l'ouverture de l'ampoule (1),
dans lequel l'appareil d'ouverture (8) est logé de manière mobile contre l'aide au positionnement (6) et/ou l'aide au positionnement (6) est logée de manière mobile contre l'appareil d'ouverture (8), et
au moins un élément d'accumulation d'énergie déformable élastiquement (9) pour l'accumulation d'énergie élastique, dans lequel un mouvement de l'appareil d'ouverture (8) et/ou un mouvement de l'aide au positionnement (6) est à entraîner avec l'énergie élastique de l'au moins un élément d'accumulation d'énergie déformable élastiquement (9) et l'ampoule (1) est à ouvrir par le mouvement de l'aide au positionnement (6) avec l'ampoule (1) et l'appareil d'ouverture (8) l'un par rapport à l'autre, dans lequel le dispositif d'ouverture présente un réservoir (2) pour la réception de l'ampoule (1) et le réservoir (2) présente un espace (7) pour la réception d'une tête d'ampoule (4) de l'ampoule (1), **caractérisé en ce que** cet espace (7) possède au moins une paroi déformable mécaniquement (12) sur laquelle la tête d'ampoule (4) repose ou se trouve à un écart proche par rapport à celle-ci, dans lequel l'appareil d'ouverture (8) entraîné par l'énergie élastique déforme la paroi déformable mécaniquement (12) de telle sorte que la tête d'ampoule (4) se casse ou s'ouvre.

2. Dispositif d'ouverture selon la revendication 1, **caractérisé en ce que** l'aide au positionnement (6) est disposée à l'intérieur du réservoir (2).

3. Dispositif d'ouverture selon une des revendications précédentes, **caractérisé en ce que**
l'aide au positionnement (6) est un support d'ampoule qui est disposé sur le réservoir (2) et auquel l'ampoule (1) est fixée ou peut être fixée.

4. Dispositif d'ouverture selon une des revendications précédentes, **caractérisé en ce que**
au moins un élément de tamis (22) ou élément de filtre (22) perméable pour des liquides est prévu, lequel est disposé dans l'espace (7) pour la retenue de la tête d'ampoule (4) et/ou de fragments de l'ampoule (1) en dessous de la tête d'ampoule (4).

5. Dispositif d'ouverture selon une des revendications précédentes, **caractérisé en ce que**
l'appareil d'ouverture (8) est un poussoir (8) logé de manière mobile linéairement et le mouvement linéaire du poussoir (8) est à entraîner avec l'énergie élastique de l'au moins un élément d'accumulation d'énergie déformable élastiquement (9).

6. Dispositif d'ouverture selon une des revendications précédentes, **caractérisé en ce que**
l'au moins un élément d'accumulation d'énergie déformable élastiquement (9) est au moins un ressort (9).

7. Dispositif d'ouverture selon la revendication 6, **caractérisé en ce que** l'au moins un ressort (9) est guidé avec un guide de ressort de sorte que la force de ressort élastique de l'au moins un ressort (9) agit dans une direction prédéfinie par le guide de ressort.

8. Dispositif d'ouverture selon une des revendications précédentes, **caractérisé en ce que**
l'au moins un élément d'accumulation d'énergie déformable élastiquement (9) est tendu et est bloqué à l'état tendu par au moins une attache mécanique amovible (16) de sorte qu'une libération de l'énergie élastique de l'au moins un élément d'accumulation d'énergie (9) est empêchée par l'au moins une attache (16).

9. Dispositif d'ouverture selon une des revendications précédentes, **caractérisé en ce que**
un logement en forme de cuve (23) pour le liquide monomère est prévu en dessous de l'aide au positionnement (6).

10. Système de mélange sous vide pour la fabrication d'un ciment présentant un dispositif d'ouverture selon une des revendications 1 à 9, une cartouche de mélange dans laquelle une poudre de ciment est contenue, un récipient de liquide monomère (1) qui est une ampoule et qui est positionné par l'aide au positionnement (6), et un moyen de conduite (20) qui connecte la cartouche de mélange à une ouverture dans la région de l'ampoule (1) ou du logement en forme de cuve (23) pour le liquide monomère.

11. Système de mélange sous vide selon la revendication 10, **caractérisé en ce que**
le système de mélange sous vide présente un pied (24) qui porte et connecte les uns aux autres la cartouche de mélange, le moyen de conduite (20) et le dispositif d'ouverture, dans lequel la cartouche de mélange est de préférence connectée au pied (24) de manière amovible.

12. Procédé d'ouverture d'un récipient de liquide monomère (1) qui est une ampoule avec un dispositif d'ouverture selon une des revendications 1 à 10, dans lequel
de l'énergie élastique est prélevée de l'au moins un élément d'accumulation d'énergie déformé élastiquement (9), dans lequel l'appareil d'ouverture (8) et/ou l'aide au positionnement (6) est entraîné(e) avec l'énergie élastique de sorte que l'appareil d'ouverture (8) et/ou l'aide au positionnement (6) exercent une force sur l'ampoule (1), et l'ampoule (1) est ouverte avec la force exercée de la sorte.

13. Procédé selon la revendication 12, **caractérisé en ce que**
au moins un élément d'attache (16) maintient l'au moins un élément d'accumulation d'énergie (9) dans l'état déformé élastiquement, dans lequel l'au moins un élément d'attache (16) est détaché pour prélever l'énergie élastique de l'au moins un élément d'accumulation d'énergie (9).

14. Procédé selon une des revendications 12 ou 13, **caractérisé en ce que**
un ressort tendu (9) en tant qu'élément d'accumulation d'énergie (9) pousse sur un poussoir (8) en tant qu'appareil d'ouverture (8) et/ou un ressort tendu (9) en tant qu'élément d'accumulation d'énergie (9) pousse sur l'aide au positionnement (6) ou sur l'ampoule (1) en tant que récipient de liquide monomère (1), dans lequel le poussoir (8) est poussé contre l'ampoule (1) avec le ressort (9) et/ou l'ampoule (1) est poussée contre le poussoir (8), dans lequel la force qui est exercée de ce fait sur l'ampoule (1) est utilisée pour ouvrir l'ampoule (1).
